(19) Europäisches Patentamt / European Patent Office / Office européen des brevets

(11) **EP 2 086 344 B1**

(12) # EUROPÄISCHE PATENTSCHRIFT

(45) Veröffentlichungstag und Bekanntmachung des Hinweises auf die Patenterteilung:
**28.09.2022  Patentblatt 2022/39**

(21) Anmeldenummer: **07817566.8**

(22) Anmeldetag: **24.09.2007**

(51) Internationale Patentklassifikation (IPC):
*A23J 1/14* *(2006.01)*  *A61K 8/64* *(2006.01)*
*A61K 47/42* *(2017.01)*  *C09H 3/00* *(2006.01)*
*A23K 20/147* *(2016.01)*  *A23L 13/40* *(2016.01)*
*A23L 23/00* *(2016.01)*  *A23L 27/60* *(2016.01)*
*A23L 33/185* *(2016.01)*  *A61Q 19/00* *(2006.01)*

(52) Gemeinsame Patentklassifikation (CPC):
**A61K 8/645; A23J 1/14; A23K 20/147;
A23L 13/426; A23L 23/00; A23L 27/60;
A23L 33/185; A61Q 19/00**

(86) Internationale Anmeldenummer:
**PCT/DE2007/001724**

(87) Internationale Veröffentlichungsnummer:
**WO 2008/049385 (02.05.2008 Gazette 2008/18)**

(54) **VERFAHREN ZUM ERHALT VON LEGUMINOSENPROTEINFRAKTIONEN MITTLEREN MOLEKULARGEWICHTS**

PROCESS FOR OBTAINING LEGUMINOUS PROTEIN FRACTIONS OF MODERATE MOLECULAR WEIGHT

PROCÉDÉ D'OBTENTION DE FRACTIONS PROTÉIQUES DE LÉGUMINEUSE D'UN POIDS MOLÉCULAIRE MOYEN

(84) Benannte Vertragsstaaten:
**AT BE BG CH CY CZ DE DK EE ES FI FR GB GR
HU IE IS IT LI LT LU LV MC MT NL PL PT RO SE
SI SK TR**

(30) Priorität: **26.10.2006   DE 102006050619**

(43) Veröffentlichungstag der Anmeldung:
**12.08.2009   Patentblatt 2009/33**

(73) Patentinhaber: **Emsland-Stärke GmbH
49824 Emlichheim (DE)**

(72) Erfinder:
- **LOTZ, Martin
  49824 Emlichheim (DE)**
- **EGGENGOOR, Gerold
  49849 Wilsum (DE)**

(74) Vertreter: **Neidl-Stippler, Cornelia
Neidl-Stippler
Patentanwaltskanzlei
Rauchstrasse 2
81679 München (DE)**

(56) Entgegenhaltungen:
**EP-A- 1 400 537     WO-A-2007/017572
DD-A1- 214 527     DE-A1- 2 922 247
FR-A- 2 889 417**

- O'KANE ET AL.: "Characterization of Pea Vicilin. 1. Denoting Convicilin as the alpha-Subunit of the Pisum Vicilin Family" JOURNAL OF AGRICULTURAL AND FOOD CHEMISTRY, Bd. 52, Nr. 10, 2004, Seiten 3141-3148, XP002467149
- CHAKRABORTY ET AL.: "Ultracentrifugation of Salt-soluble Proteins in Ten Legume Species" JOURNAL OF THE SCIENCE OF FOOD AND AGRICULTURE, Bd. 30, Nr. 8, 1979, Seiten 766-771, XP008088079
- SCHMANDKE ET AL.: "Über den Einfluss des leicht- und schwerlöslichen Anteils von Vicia-faba-Proteinisolat auf die funktionellen Eigenschaften von Proteinfasern" DIE NAHRUNG, Bd. 25, Nr. 4, 1981, Seiten 379-389, XP002467150

- **DATABASE WPI Week 199646 Derwent Publications Ltd., London, GB; AN 1996-462955 XP002380067 & RU 2 054 265 C1 (BOGRACHEVA T YA) 20. Februar 1996 (1996-02-20)**

**Beschreibung**

Die Erfindung betrifft ein Verfahren nach dem Oberbegriff des Patentanspruches 1 Sie bezieht sich also auf die Gewinnung von pflanzlichen Proteinen-Proteine sind lebenswichtige chemische Verbindungen für die gesamte lebende Welt, meist mit biochemischer Funktion als Enzyme, aber auch als Speicherstoffe (Speicherproteine), sozusagen als Rohstoffreservoir für lebenswichtige Vorgänge, v.a. bei Wachstum bzw. Vermehrung. Proteine sind durch ihre Molekülgrösse, ihre Zusammensetzung sowie ihre Sekundarund Tertiarstruktur gekennzeichnet. Von Proteinen oder Eiweiss spricht man dann, wenn der chemische Aufbau ausschließlich aus Aminosäuren besteht und die Kettenlänge ca. 30 Aminosäuren und mehr beträgt. Bei kürzeren Ketten spricht man im allgemeinen von Peptiden, ohne dass diese Abgrenzung genau definiert wäre. Sie ist eher willkürlich und für bestimmte Situationen nützlich. Proteine werden von einzelligen Lebewesen, z.B. Bakterien und Hefen, Pflanzen, aber auch von Tieren erzeugt. Für die menschliche und tierische Ernahrung und Gesundheit sind sie unverzichtbar. Neben ihren chemischen, nutritiven und biochemischen Eigenschaften haben Proteine auch sogenannte funktionale Eigenschaften, von denen die Wasseraufnahmekapazität, Verdaulichkeit, Löslichkeit in Wasser, die Bildung und Stabilisierung von Schaum sowie die Emulsionsfähigkeit für ihre technische Anwendbarkeit herausragenden Merkmale sind, die auch durch die tertiäre und sekundäre Struktur bestimmt sind. Dadurch finden Eiweisse auch in der Technik breite Anwendung, u.a. als Kleber, Emulgatoren, Andickungsmittel. Die tertiäre Struktur wird durch Hitzeeinwirkung oder aber durch Einwirkung von Säuren oder Laugen schwerwiegend und häufig irreversibel gestört.

Die Sekundärstruktur wird durch Spaltung der Proteine, wie sie durch Enzyme oder aber stark alkalische oder saure Medien erfolgt, zerstört. Es ist somit wesentlich, funktionale Eigenschaften der Proteine während der Isolation derselben aufrecht zu erhalten.

Stand der Technik ist, tierische sowohl als auch pflanzliche Proteine zu isolieren. Sie werden bereits vielfältig angewendet, z.B. in Lebensmitteln (Tofu), Futtermitteln, der Pharmazie und in technischen Bereichen (Eiweißkleber od. dgl.) Die Bedeutung der Proteine für Ernährung, Lebensmitteltechnologie, z.B. als Schaummittel, Emulgator, Strukturgeber bzw. Texturierer (z.B. Gelatine für Gummibärchen und Tortenguss), Tierfutter, Kosmetika und Arzneimittel ist einzigartig und kann durch keine andere Stoffgruppe ersetzt werden. Am einfachsten sind funktionelle Proteine, die gute Wasserlöslichkeit und Emulsionsfähigkeit aufweisen, aus Milch oder Eiern durch wenig belastende Prozesse (keine starken pH-Wert-Änderungen, keine hohe Erhitzung) erhältlich.

Problematisch ist, dass insbesondere tierische Proteine häufig allergische Reaktionen auslösen.

Die sehr häufig industriell eingesetzten Kuhmilchproteine sind bei vielen Verbrauchern, die eine Lactoseintoleranz oder eine Allergie gegen Kuhmilchproteine aufweisen, gefürchtet. Tierische Proteine haben noch dazu immer den Nachteil, dass sie Träger von Krankheiten (wie BSE, HIV oder aber Vogelgrippe) tragen oder pathogen sein können. Die Verwendung tierischer Proteine hat auch den Nachteil, dass diese in vielen Bevölkerungsgruppen aus ethischen Gründen nicht akzeptiert werden. Hautcremes auf Collagenbasis etwa sind aus diesem Grund im asiatischen und moslemischen Kulturkreis verpönt, führen aber auch in unseren Kulturen zu Allergien. Auch sind tierische Proteine in aller Regel teurer als pflanzliche Proteine, da die Nachhaltigkeit pflanzlicher Proteine wesentlich höher als bei tierischen Proteinen ist - sie sind preiswerter zu produzieren und außerdem für eine vegetarische oder andere Diät, wie bspw. eine purinarme Diät oder Ernährung besonders geeignet. Dies macht es sinnvoll, pflanzliche Proteine näher zu untersuchen.

Pflanzenproteine, insbesondere die Leguminosenproteine, wie Erbsenproteine und Ackerbohnenprotein vermeiden viele der oben genannten Nachteile der tierischen Proteine. Viele sind wenig bis nicht allergen - d.h. z.B. nicht auf der Allergenliste der EU geführt, als pflanzliches Protein in allen Kulturen akzeptiert und aufgrund der Kultur bestimmter Pflanzen, wie von Erbsen oder Bohnen, ist die Sicherstellung gentechnikfreier Produkte (non-GMO-Zertifikate) und von Bio-Produkten möglich.

Bei allen industriell verwendeten tierischen Proteinen wie etwa Milch- und Molkeproteine, Gelatine, Hühnereiweiß, Collagen usw. sind im technischen Einsatz im allgemeinen - d.h. in der Lebensmitteltechnologie, der Technik u. dgl. fast ausschließlich Milchproteine, v.a. Casein und seine Salze ( z.B. als Etikettierleim für Flaschenetiketten), Hühnereiweiß als Volleiweiß oder Eiklar sowie Eigelb und aus Schlachtabfällen isolierte Proteine, wie Gelatine, Knochenleim und Collagen (z.B. in der Kosmetik oder als Kleber).

Pflanzliche Proteine sind bis heute nur aus wenigen Pflanzen in der täglichen Anwendung verbreitet. Gebräuchliche isolierte Leguminosenproteine stammen von Leguminosen, wie Soja, Erbsen, Lupinen, Linsen. In größerem Umfang sind es als Isolate nur Soja- und Weizenprotein, der sogenannte Gluten. Gluten ist ein problematisches Eiweiß, da viele Menschen unter einer Glutenallergie leiden (Zöliakie) und ein erheblicher Bedarf an glutenfreiem pflanzlichem Eiweiß besteht. Unfraktioniertes Sojaprotein wie auch viele andere Leguminosenproteine, das in großem Umfang als Austauschstoff für tierisches Eiweiß sowie als Tierfuttermittel weit verbreitet ist, ist nicht unumstritten, da es hormonaktive Substanzen enthält. Typisch für Leguminosen sind Genistein und Daidzein; Anti-thyroidisoflavone, welche die Schilddrüsenaktivität hemmen. Genistein ist 5,7,4'Trihydroyisoflavon mit einem Molekulargewicht von 270,24 Dalton. Weiterhin enthält Soja Proteaseinhibitoren, Phytinsäure, Lectine und Sojatoxin.

Weitere isolierte Leguminosenproteine stammen aus Raps, Lupinen und Linsen. Die Qualität vieler kommerziell erhältlicher isolierter Leguminosenproteine, insbesondere der Erbsenproteine, ist nicht zufriedenstellend. Die Gründe hierfür sind verschieden. Bei Raps und dgl. ist es vor allem der Fettgehalt der Proteine, der zu Ranzigkeit der Eiweiße und der daraus hergestellten Produkte führt. Dies gilt auch für Futterproteine, da ranzig gewordenes pflanzliches Fett in das tierische Fleisch eingelagert wird und ihm den typischen ranzigen Geschmack gibt. Bei den z.Zt. erhältlichen pflanzlichen Proteinen ist auch der starke Eigengeschmack ein Problem, wie z.B. bei Sojaprotein, wodurch viele Anwendungen nicht möglich sind oder die Einsatzmenge limitiert ist, was ebenfalls oft nachteilig ist. Für Nahrungsmittelzwecke ist zu beachten, dass die Leguminosenproteine nicht vollwertig sind, d.h. dass sie nicht alle Aminosäuren, insbesondere die B sog. essen-tiellen Aminosäuren, die der menschliche Körper nicht selbst herstellen kann und daher extern zugeführt bekommen muss, enthalten. Die Wertigkeit von Legumino-senproteinen ist geringer als die von tierischen Proteinen - aber dennoch gut. Für die Herstellung von Leguminosenprotein aus Fruchtwasser, das durch Pressen der entsprechenden Pflanzenteile, insbesondere Früchte, oder durch Flüssig-Extraktion aus zerkleinerten Pflanzenteilen gewonnen wird, werden technisch prinzipiell zwei Verfahren eingesetzt:

I. Wärmekoagulation der Proteine im Fruchtwasser unter Inaktivierung der Enzyme oder
II. Ausfällung der Proteine aus dem Fruchtwasser bei saurem pH oder Kombination aus beidem.

2. Abtrennung des ausgefällten Proteins

*Verfahren zur Gewinnung von Leguminosenproteinen sind aus* DE2922247 A1 *bekannt, die eine Fällung von Leguminosenproteinen* am *isoelektrischen Punkt lehrt* O'Kane et. al. lehrt die wissenschaftliche Charakterisierung von Erbsenproteinen IM JOURNAL OF AGRICULTURAL AND FOOD CHEMISTRY 52, Nr. 10, 2004, Seiten 3141-3148*:* Chakraborty et. al. beschreibt in: JOURNAL OF THE SCIENCE OF FOOD ANDAGRICULTURE" 30. Nr. 8, 1979, Seiten 766 - 711 die Ultrazentrifugationstrennung von salzlöslichen Proteinen von 10 Leguminosen-früchten *und* Schmandke et. al. beschreiben in DIE NAHRUNG, Bd. 25, Nr. 4, 1981, S. 379 - 389 *die Einflüsse von Faba-Bohnen-Proteinisolaten auf Proteinfasern, welche als texturierte vegetabile Produkte verwendet werden sollen,* DD214527 *erläutert Leguminosenproteinfällung und eine Proteinbehandlung bei hohen Temperaturen, was die Funktionalität der so hergestellten Proteine negativ beeinflusst.*

Unter Pflanzenteilen werden im Zusammenhang mit der Erfindung die Samen von Leguminosen, nämlich Ackerbohnen und Erbsen verstanden. Unter Fruchtwasser werden hier sowohl aus den Samen ausgepresste Säfte als auch durch Extraktion mit wässrigen Medien aus den Samen gewonnene Lösungen von Leguminosenproteinen verstanden, die dann durchzuführen ist, wenn das Pflanzenteil keinen ausreichenden Flüssigkeitsgehalt besitzt oder aber um verbliebenes Protein zu mobilisieren.

Bei der klassischen Hitzefällung der Proteine aus dem Fruchtwasser entsteht ein schwerlösliches Produkt unzureichender Funktionalität, das schlecht verdaulich ist, starken Nebengeschmack hat und noch dazu mit Schadstoffen behaftet ist. Bei höherer Temperatur wird das Protein übermäßig geschädigt und seine wertvollen Eigenschaften, die es für die Lebensmittelanwendung so nützlich machen werden zunehmend zerstört: neutraler Geschmack, helle Farbe, Löslichkeit, alle anderen Funktionalitäten ebenfalls, die Struktur wird verhornt und die Verdaulichkeit nimmt ab. Dies gilt insbesondere für das sog. Texturized Vegetable Protein (TVP), ein Sojaprotein, das als Ersatzstoff für Fleisch eingesetzt wird - aufgrund der starken Temperaturbelastung bei der Herstellung ist es schlecht verdaulich und hat einen extremen bohnenartigen Geschmack. Ein weiterer Nachteil der bislang bekannten Verfahren zur Isolation von Leguminosenproteinen ist, dass antinutritive Stoffe nicht entfernt werden. Dies geschieht durch gezielte Auswaschung mit viel Waschwasser bei z.T. nur wenig Trockensubstanz, da diese oft nur eine geringe Löslichkeit besitzen, oder aber man benutzt teure Lösungsmittel, die bspw. Isoflavone lösen (Alkohol) die wiedergewonnen und aufgearbeitet werden müssen. Beide Verfahren sind sehr aufwendig.

Die so hergestellten bekannten Leguminosenproteine weisen dazu geringe Funktionalität und auch aufgrund ihrer hohen Denaturierung schlechte physiologische Verwertbarkeit auf, sind also schlecht zu verdauen und eignen sich schlecht bis gar nicht als Milchproteinersatzstoffe. Ähnliches gilt für die Proteine anderer Pflanzen, wie Weizen, Raps, Soja etc., bei denen schädliche Nebenstoffe vorliegen.

Typische weitere Negativstoffe, die als Begleitstoffe in gefällten Leguminosenproteinen auftreten, sind bspw.: Trypsininhibitoren, Proteine, welche das Eiweißverdauungsenzym Trypsin und damit die Verdauung hemmen. Trypsininhibitor kann nur durch gezielte Deaktivierung unschädlich gemacht werden, hier durch die Hitzebehandlung von um 70 °C.

Ferner können u.a. auftreten: Phytinsäure (komplexiert Ca-Ionen), hindert Ca-Aufnahme, Lectin, und andere Enzymhemmer als ein Verdauungshemmer, Tannine/Gerbsäuren, hindern die Verdauung, die Eisenaufnahme und inaktivieren Verdauungsenzyme; Proteaseinhibitoren, Polyphenole und spezielle Zucker, wie Leguminosenzucker, die bspw. zu Diarrhöe führen können. Ferner sind für Leguminosen hormonartige Substanzen typisch, meist Isoflavone. Die Abtrennung oder Unschädlichmachung derselben geschieht heute nur teilweise - bspw. durch Auswaschen der Proteine oder aufwendige und teure enzymatische Behandlung. Viele Leguminosenproteine, die durch Hitzefällung einer Proteinlösung im alkalischen Milieu gewonnen werden, (wozu übrigens auch die ggf. notwendige Pasteurisierung zählt), sind noch dazu durch einen hohen Gehalt an Lysinoalanin gekennzeichnet, einem antinutritiven Kondensationsprodukt, dessen Gehalt grundsätzlich so gering wie nur möglich sein soll.

Es ist Aufgabe der Erfindung, *industrielle Verfahren zur Herstellung von Leguminosenproteinen* aus Ackerbohnen und Erbsen mit besserer Funktionalität bereitzustellen, die Nachteile bekannter Leguminosenproteine vermeiden.

Die Aufgabe wird erfindungsgemäss durch ein Verfahren mit den Merkmalen des Patentanspruches 1 gelöst. Vorteilhafte Weiterbildungen ergeben sich aus den abhängigen Ansprüchen.

Erfindungsgemäss wird also durch gezielte Fraktionierung des Proteins aus wässriger Lösung mit proteinschonenden, überraschend einfachen Verfahren eine Proteinfraktion lebensmitteltauglicher Qualität mit Funktionalität erhalten. Wesentlich ist, dass die Fraktionierung als Trenntechnik für verschiedene Proteine eingesetzt wird. Die Fraktionierung durch gezielte Einstellung von pH-Wert und Temperatur ist ein effizientes und preiswertes Verfahren, was eine einfache überraschend gezielte Proteinfraktionierung im technischen Grossmassstab ermöglicht. Sie ist auch durch stufenweise Membranfiltration oder stufenweise Fällung mit Lösungsmitteln oder fraktioniertes Aussalzen möglich, aber erheblich aufwendiger. Es ist häufig günstig, die mechanische Abtrennung mit Dekantern durchzuführen, die schnell grosse Mengen Material in Feststoffe und Überlauf kontinuierlich trennen können.

Als hochwertige Leguminosenproteine (viele essentielle Aminosäuren) eignen sich Erbsenproteine und Ackerbohnenproteine.

Die mit dem gemäß dem erfindungsgemäßen Verfahren hergestellten Leguminosenproteinfraktionen eignen sich besonders als Lebensmittel, Nahrungsmittelzusatz, Arzneimittelzusatz, Tierfutter, in der Kosmetik, als technisches Eiweiß, als Kleber, da sie einerseits in ausreichender Menge vorliegt, andererseits eine ausreichende Funktionalität durch Löslichkeit in Wasser aufweist.

Eine gezielte Fraktionierung des Leguminosenproteins, wie es bspw. im Erbsenwasserextrakt vorliegt, erfolgt mit der Zielsetzung, preiswert aus dem Fruchtsaft zu isolieren. Erfindungsgemäß geht man so vor, dass von Verfahrensschritt zu Verfahrensschritt die Fällungsbedingungen verschärft werden, um so jeweils die Fraktion mit den Proteinen, die das nächst niedrigere Molekulargewicht besitzen, isolieren zu können. Man nutzt dabei das deutlich verschiedene Verhalten der Leguminosenproteinfraktionen abhängig von verschiedenen Kombinationen aus Temperatur und pH-Wert.

Das erfindungsgemäße Verfahren weist die Schritte gemäß dem Anspruch 1 auf.

Stufe 1

Der erste Schritt dient der Abtrennung großer Proteine; er trennt mechanisch eine Fraktion des Leguminosenproteins nach einer ersten Säurefällung, das "Sedimentprotein", ab. Dieses ist hochmolekular und ein reines Protein. Protein niedrigeren Molekulargewichts verbleibt im Überstand. Dadurch wird eine aufwendige Abtrennung von unerwünschten Schadstoffen durch Adsorption in dieser Fraktion, vermieden. Ein so isoliertes Erbsenprotein besitzt einen Proteingehalt von 90 % (N*6,25), ist hochmolekular und daher wenig löslich und funktionell..

Die Abtrennung dieser Proteine mit einem Molekulargewicht von > ca. 116 kD kann man bspw. einfach erreichen durch (die aufwendigen Verfahren, wie Membranfiltration, werden hier wegen des höheren Aufwandes als untergeordnet betrachten):

- einfaches fraktionierendes Zentrifugieren des reinen Fruchtsaftes, der von sich aus einen leicht sauren pH-Wert besitzt. Hierbei wird am wenigsten Protein abgetrennt, dafür aber "sortenrein"
  oder
- Einstellen eines sauren pH-Wertes zwischen 5 und 7 und Säurefällung der großen Proteine, wobei das Ausgefällte dann mechanisch, bspw. durch Zentrifugation, abgetrennt wird. Wichtig ist, dass nicht zuviel Ausfällung (was die Ausbeute erniedrigt), und nicht zu wenig (was die Proteinreinheit der anderen Fraktionen und andere Qualitätsparameter verschlechtert) auftritt.

Ein besonderer Vorteil dieser Verfahren ist die außerordentliche Einfachheit, dies auch bezüglich Maschinen, Materialien und Energieverbrauch.

Stufe 2

Hier wird eine Zielproteinfraktion mittleren Molekulargewichts durch Einstellen eines zur Fällung geeigneten pH-Wertes in Kombination mit einer Warmefallung aus dem Überstand der Stufe 1 isoliert (selbstverständlich können auch aufwendigere Verfahren, wie die Membranfiltration, eingesetzt werden). Dazu wird ein pH-Wert bevorzugt nahe des isoelektrischen Punktes des Proteins gewählt, in Kombination mit Erhöhung der Temperatur über Raumtemperatur hinaus.

Dies entspricht wiederum den Bedingungen der Fällung aus dem Sauren bei erhöhter Temperatur - es ist bei einem pH von ca. 2 bis 6 und 50 - 85 °C zugänglich.

Überraschenderweise kann durch die Varianten des erfindungsgemässen Verfahrens gemäss Unteransprüchen 2, 3 und 4, die keine teuren oder komplizierten Verfahren oder Prozessstufen benötigt und sehr schnell ist, mit einer Kombination einfacher Verfahrensschritte das geforderte Ziel erreicht werden, insbesondere sind die notwendigen Apparaturen auch noch preiswert in der Anschaffung und im Betrieb.

Sehr vorteilhaft ist, dass keine chemischen Zusätze im Verfahren angewandt werden müssen, wie z.B. Enzyme, Desinfektionsmittel, Aufheller.

Überraschend ist auch hinsichtlich der Produktmerkmale bzw. -eigenschaften, dass durch die Fraktionierung nicht nur Proteine mit einem besonderen Molekulargewichtsbereich isoliert werden, sondern auch eine Fraktion, die alle geforderten Eigenschaften besitzt: hoch nutritiv, neutrale Farbe, (fast) kein Rohstoffgeschmack, geringe Lysinoalanin-Gehalte sowie technologische Funktionalitäten, wie Wasserbindung und Öfbindung (Emulgator). Dies ist besonders überraschend, da die zuerst gefällte Proteinfraktion bei Erbsen aufgrund des hohen Molekulargewicht fast wasserunlöslich ist. Die Bohnen und Erbsenproteine der 2. Fraktion sind sehr wohl löslich in Form einer Kurve mit einem breiten Minimum, von 15 bis 70 % Löslichkeit, abhängig vom pH-Wert.

Nachstehend wird die Erfindung nunmehr anhand von Ausführungsbeispielen, auf die sie keineswegs eingeschränkt ist, näher erläutert.

Die erfindungsgemäss hergestellte Leguminosenproteinfraktion vermeidet Nachteile bekannter Leguminosenproteine (Farbe, Bitterkeit, Allergenität, antinutritive Produkte, Eigengeschmack, Funktionslosigkeit) durch Auswaschen. Es kann auch eine fraktionierte Fällung von 2 Proteinfraktionen durchgeführt werden. Antinutritiv könnten einige Enzyme, sein. Diese Enzyme haben ein Molekulargewicht in der Grössenordnung von 2 kD bis 9 kD, so dass diese nicht in der hier beschriebenen Fraktion in nennenswerten Mengen enthalten sind. Darüber hinaus bewirkt die Wärmebehandlung eine Denaturierung und Inaktivierung von Enzymen.

Schadstoffe: Als Schadstoffe kommen ausser den "normalen" Umweltgiften wie Schwermetalle und Pestizide hormonaktive Substanzen, wie Isoflavone und AntiThyroid Isoflavone, sowie spezifische Toxine, vor. Deren Abtrennung erfolgt wie oben beschrieben. Ein allergenes Potential der erfindungsgemässen Leguminosenproteinfraktionen mit einem Molekulargewicht von 14 - 97 kD ist unbekannt und sie eignen sich daher auch für die Produktion spezieller Allergenfreier pflanzlicher Spezialnahrung und Kosmetika.

Messung der Emulgierkapezität:

25 g Protein werden in 100 g Wasser mit einem Ultraturrax suspendiert. Danach gibt man unter weiterem Dispergieren langsam und portionsweise Öl dazu (z.B. Sonnenblumenkernöl, Rapsöl, Olivenöl usw.) bis die Emulsion bricht. Man gibt die Ölbindungsoder Emulsionskapazität im Verhältnis der 3 Stoffe an mit der maximalen Ölbindung. 1 : 4: 6 bedeutet, dass eine Mischung aus 1 Teil Protein und 4 Teilen Wasser 6 Teile Öl binden können, d.h. nach Zugabe von mehr als 150 g Öl in o. g. Suspension bricht die Testemulsion.

Wasserbindekapazitätsbestimmung:

5 g Protein werden in 95 g Wasser eingewogen und die Suspension 1 h gerührt. Danach wird zentrifugiert ( 20 min., 3.500 g), der Überstand vorsichtig abgegossen (u.U. den letzten Rest abpipettieren) und das nasse Protein ausgewogen.

$$(\text{Nassgewicht} - \text{Trockengewicht})/\text{Trockengewicht} = \text{Wasserbindekapazität}$$

Erbsenprotein (Verfahren nur bezüglich der in Anspruch 1 angegebenen Parameter Teil der Erfindung)

Für Erbsen ist wegen des geringen Wassergehalts zunächst eine Quellung von geschälten oder ungeschälten Erbsen bei einem pH-Wert in der Größenordnung von pH 2 - 10 über einen ausreichend langen Zeitraum im Bereich von 10 min bis mehreren Stunden vorgesehen, wobei die Lösung anschließend gepresst oder zentrifugiert, der Feststoff abgetrennt und der flüssige Überstand zur Eiweißisolation verwendet wird.

Dabei gilt:

1. Stufe:

Einstellen von pH 4,0 - 7,0 in der Extraktlösung, dadurch Ausfällen einer Erbsenproteinfraktion mit einem Molekulargewicht von ca. 100 - 600 kD bei Raumtemperatur und separieren derselben.

2. Stufe:

Einstellen des pH der Lösung auf 4,0 bis 6,0 und 50 - 85 °C (nahe isoelektrischer Punkt), der entstehende weiße Niederschlag (pH 4 - 6 zur Fällung) wird bei oben genannten Prozessbedingungen mit einer Dekanterzentrifuge mechanisch abgetrennt. Proteingehalt des Rohprodukts ist ca. 75 % in Trockensubstanz. Die Parameter gewährleisten auch mikrobiologische Reinheit des Produkts, es ist also keine zusätzliche Pasteurisierung notwendig. Keine hohe bis sehr hohe Temperatur und saures Milieu, daher wenig Lysinoalanin.

Reinigungsschritte:

Danach erfolgt die Reinigung des Proteins zu Isolatqualität, d.i. > 85 % Protein in Trockensubstanz. Prozessparameter: Leitungswasser, kalt (Raumtemperatur) oder heiß (bevorzugt 50 °C bis 80 °C), pH neutral oder angesäuert auf pH 4 bis 6.
Waschwassermenge dieselbe bis die doppelte Menge wie der Produktzulauf zur Dekanterzentrifuge. Beispielsweise kann die Ausführung in zwei Stufen erfolgen, d.h. durch zwei in Reihe geschaltete Dekanter, wobei vor jedem Dekanter die Hälfte des Waschwassers zugeführt wird oder im Gegenstrom, d.h. das gesamte Waschwasser wird vor dem 2. Dekanter zugeführt und im Oberlauf des 1. Dekanters aus dem Prozess ausgeschleust.
Es ist besonders darauf zu achten, dass bei höherer Temperatur das Protein übermäßig geschädigt und seine wertvollen Eigenschaften, die es für die Lebensmittelanwendung so nützlich machen, zunehmend zerstört werden: neutraler Geschmack, helle Farbe, Löslichkeit, alle anderen Funktionalitäten ebenfalls, die Struktur wird verhornt und die Verdaulichkeit nimmt ab. Übrig bleibt im Leguminosenfruchtwasser die Proteinfraktion, die auch unter diesen verschärften Bedingungen löslich bleibt. Weniger scharfe Bedingungen verringern nur unnötig die Ausbeute.
Gemäß SDS-PAGE betragen die Molekulargewichte der verschiedenen Erbsenproteine in der hohen Fraktion ca. 100 - 600 kD und in der mittleren Fraktion ca. 14 - 97 kD, wobei das Maximum der Proteine bei 20 und verschmiert von 36,5 bis 97 kD liegt, die restlichen Banden sind vernachlässigbar.
Das Produkt der mittleren Fraktion wies folgende Eigenschaften auf:

max. 1% Stärke

max. 1% Zucker

max. 1% Rohfaser

Isoelektrischen Punkt von etwa 4,3

pH-Wert von 4 - 6

Emulgierkapazität von 1:4:10 - 23 (Messung der Emulgierkapazität s.o.)

Löslichkeit 15 - 70 % in Wasser bei Raumtemperatur und auch in heißem Wasser, abhängig vom pH-Wert)

Wasserbindekapazität: 1:4 bis 1:5 (Testverfahren: s.o.)

Die Aminosäurezusammensetzung der so erhaltenen Erbseneiweißfraktion mit einem Molekulargewicht zwischen 20 und 97 kD betrug:

| | |
|---|---|
| Ala | 3,7 - 4,2 |
| Arg | 7,1 - 7,3 |
| Asp | 10,1 - 10,2 |
| Cys | 0,9 - 1,0 |
| Glu | 14,2 - 14,7 |
| Gly | 3,5 - 3,9 |
| His | 2,0 - 2,2 |
| Ile | 4,1 - 4,6 |
| Leu | 7,7-8,1 |

(fortgesetzt)

| Met | 0,9 - 1,2 |
|-----|-----------|
| Phe | 5,0 - 5,5 |
| Pro | 3,9 - 4,1 |
| Ser | 4,2 - 4,6 |
| Ihr | 3,3 - 3,6 |
| Try | 0,8-1,0 |
| Tyr | 4,0 - 4,2 |
| Val | 4,5 - 5,4 |
| Lys | 6,3 - 7,6 |

Essentielle Aminosäuren sind unterstrichen. Der Gesamtgehalt essentieller Aminosäuren ist 32,6 % bis 36,8 %, Gesamtaminosäuren in der Trockensubstanz 87,1 %, in OS 82,2 %, Rohprotein (N*6,25) 85,6 % in der Trockensubstanz. Die Schwankungen sind typisch für Naturprodukte Mit dieser Erbseneiweißfraktion wird insbesondere für die Herstellung konfektionierter Lebensmittel ein Protein zur Verfügung gestellt, das die erwähnten technologischen Vorteile bietet.

Ackerbohnen: (Verfahren nur bezüglich der in Anspruch 1 angegebenen Parameter Teil der Erfindung)

Bei Ackerbohnen findet Isolation analog zu Erbsen statt. Im Gegensatz zu Erbsen waren für die Fällung der Fraktion mittleren Molekulargewichts die pH-Werte 4,2 - 5,3 gleichwertig. Dies kommt den isoelektrischen Punkten der beiden Hauptproteinfraktionen (pH 5,5) und (pH 4,8) recht nahe. Die mittlere Fraktion hat ein Molekulargewicht zw. 20 kD und 116, Maxima bei ca. 36 und vor allem ca. 51 (Nebenbanden bei 66, 116 kD, ganz dünn 21 und 23 kD).

Die Aminosäurezusammensetzung der so erhaltenen Bohneneiweißfraktion mit einem Molekulargewicht zwischen 20 und 116 kD betrug:

| Ala | 3,0 |
|-----|-----|
| Arg | 9,5 |
| Asp | 11,0 |
| Cys | 1,0 |
| Glu | 16,1 |
| Gly | 3,3 |
| His | 2,9 |
| Ile | 4,7 |
| Leu | 7,9 |
| Met | 0,4 |
| Phe | 5,1 |
| Pro | 6,4 |
| Ser | 4,3 |
| Thr | 3,2 |
| Try | 0,8 |
| Tyr | 4,7 |
| Val | 4,6 |
| Lys | 5,7 |

Essentielle Aminosäuren sind unterstrichen. Der Gesamtgehalt essentieller Aminosäuren ist 32,4 %.

Herstellung von Erbseneiweiß

50 kg geschälte Erbsen (Handelsware der Sorte Pisum sativum L.) wurden in einer Mühle zerkleinert und 0,5 Std. bei pH 6,0 - 8,0 gequollen. Anschließend wurde Erbsenprotein haltige Lösung durch Auspressen der gequollenen Reibsel in einem Dekanter gewonnen. Die erbsenproteinhaltige Lösung wurde auf pH 5,5 gebracht und das ausgefällte Protein als Sedimentprotein in einem Schlammseparator separiert. Der flüssige Überstand wurde sodann auf 80 °C erhitzt und auf pH 5,0 unter Ausfällung der Proteinfraktion mittleren Molekulargewichts gebracht Die so ausgefällte Proteinfraktion wurde in einem Dekanter abgetrennt Die Fraktionen wurden gewaschen und 2,5 kg Sedimentprotein und 5 kg mittleres Protein mit jeweils ca. 7 % Restfeuchtigkeit in Form eines hellen Pulvers erhalten.

### Herstellung von Ackerbohneneiweiß

Die Herstellung des Eiweißes von Ackerbohnen erfolgte wie diejenige von Erbseneiweiß.

Die so hergestellten Ackerbohnenproteine wiesen ein mittleres Molekulargewicht im Bereich von Molekulargewicht zw. 20 kD und 116, Maxima bei ca. 51 und ca. 36 auf. Sie besitzen eine Funktionalität, welche in etwa derjenigen von Milchproteinen, wie Casein, entspricht und können in ähnlicher Weise eingesetzt werden.

### Verwendung der Erbseneiweißfraktion als Emulgator in einer hypoallergenen Salatcreme (nicht Teil der Erfindung)

43,2 % Rüböl wurden mit 10 % Salzeigelb, 34,08% Wasser, 6,00 % Erbsenprotein, 1,15% NaCl, 7,2% Zucker, 0,5% Kartoffelfaser, 0,03% Paprika, 0,01 % Carotin, 0,05% weißem Pfeffer, 7,14% 10%igen Branntweinessigs und 0,64 % scharfem Senf verrührt. Es entstand eine hypoallergene Salatcreme, in der Stärke und Eiweiß, die üblicherweise als Dickungsmittel und Emulgatoren verwendet werden, vermieden werden konnten, die eine gute Emulsionsstabilität sowie Haltbarkeit aufwies und geschmacklich nicht zu beanstanden war.

### Verwendung der Erbseeiweißfraktion als Emulgator in hypoallergenem Tomatenketchup (nicht Teil der Erfindung)

30% 2-fach konzentriertes Tomatenmark, 35,4% Wasser, 9,5% 10%iget Branntweinessig, 19,00 % Zucker, 2,3% Salz, 2,5% Erbsenprotein, 0,5% Kartoffelfaser und 0,8% Zitronensäure wurden verrührt. Es wurde eine konservierungsmittelfreier Ketchup unter Austausch der sonst verwendeten Weizenstärke für Glutenallergiker erhalten.

### Kohlenhydratreduzierter Nudelteig (nicht Teil der Erfindung)

150g Vollei, 400g Erbsenprotein, 5 g Guarkernmehl, 100 g Wasser, 60g Kartoffelfasern und 6 g Salz wurden zu einem Nudelteig verknetet und in Nudeln umgeformt sowie getrocknet. Es wurde ein Produkt mit niedrigem Kohlenhydratgehalt, insbesondere mit wenig schnell absorbierbaren Kohlenhydraten erhalten, das sich für die Gewichtsreduktion sowie Diabetiker eignet.

### Proteinangereicherte Karottencremesuppe (nicht Teil der Erfindung)

300g Karotten, 200g Kartoffeln, 40g Lauchzwiebeln, 15g Petersilie, 500g Wasser, 100g Erbsenprotein, 10g Limonensaft, 250g Milch, 100 g saure Sahne, 2g schwarzer Pfeffer und 17 g Salz wurden zu einer Suppe von 1534 g verarbeitet. Diese Suppe eignet sich insbesondere als proteinangereicherte Aufbaukost.

### Kaffeeweißer (Sahneersatz) (nicht Teil der Erfindung)

82,7 % Wasser, 10% Kokosnussöl, 5% Zucker, 2,25 % wasserlösliches Erbsenprotein und 0,05 % Xanthan wurden innig verrührt. Es entstand eine geschmacklich neutrale, sahneartige Flüssigkeit, die als Sahneersatz, insbesondere als Kaffeesahne, für Milchprotein- und Lactoseallergiker einsetzbar ist.

### Rinder-Patty (nicht Teil der Erfindung)

85% Rindfleisch, mager, 10,65 % Wasser, 3% Erbsenprotein, 1,2% Salz, 0,15 % schwarzer Pfeffer wurden verknetet und daraus Fleischbällchen geformt. Hier diente das Erbsenprotein der Wasserbindung, Texturverbesserung und als Extender.

Obwohl die Erfindung anhand bevorzugter Ausführungsbeispiele erläutert wurde, ist dem Fachmann ersichtlich, dass im Rahmen der Ansprüche mannigfaltige Abwandlungen derselben möglich sind, die ebenfalls unter den Schutzumfang fallen.

## Patentansprüche

1.  Verfahren zur Herstellung von koagulierten Leguminosenproteinfraktionen ausgewählt aus Erbsen- oder Ackerbohnenproteinfraktionen eines Molekulargewichts > 14, **gekennzeichnet durch**:

Vorlegen von Fruchtsaft, hergestellt durch Abtrennen von Feststoffen aus zum Freisetzen von Fruchtsaft aus den Zellen geteilten Früchten, ggf. unter Zusatz von wässrigem Lösemittel und vollständiges oder teilweises Abtrennen des so freigesetzten Fruchtsaftes, der das gesamte Protein enthält;

Fällen einer hochmolekulargewichtigen Leguminosenfraktion, deren Hauptmenge ein Molekulargewicht von > 116 kD < 600 kD gemäß SDS-Page besitzt, durch Einstellen eines pH-Wertes im Bereich des isoelektrischen Punktes im Fruchtsaft von 5 - 7 unter Ausfällen einer Fraktion und mechanisches Abtrennen der so ausgefällten Fraktion;

und vollständigem oder teilweisem Abtrennen des so freigesetzten Fruchtsaftes, der das gesamte Protein enthält, von den Feststoffen; und

Fällen einer mittelmolekularen koagulierten Leguminosenproteinfraktion in der Wärme durch Behandeln der aus der Abtrennung der hochmolekulargewichtigen Leguminosenproteinfraktion gewonnenen Lösung bei pH 3 - 6 und zwischen 60 - 90°C, bevorzugt 65 - 80°C und Mechanisches Abtrennen der mittelmolekulargewichtigen koagulierten Leguminosenproteinfraktion mit einem Molekulargewicht zwischen 14 - 120 kD gemäß SDS-Page, deren Schwerpunkt der Molekulargewichtsverteilung zwischen 20 - 66 kD gemäß SDS-Page liegt

**2.** Verfahren nach Anspruch 1, **gekennzeichnet durch** Waschen der so erhaltenen koagulierten Leguminosenproteinfraktion.

**3.** Verfahren nach einem der vorangehenden Ansprüche, wobei das Waschen mit Wasser bei einer Temperatur von Raumtemperatur bis 80°C, bevorzugt der Temperatur der Fällung, erfolgt.

**4.** Verfahren nach Anspruch 3, wobei das Waschen bei einem pH - Wert im Bereich des isoelektrischen Punktes erfolgt

**5.** Verfahren nach einem der vorangehenden Ansprüche, **dadurch gekennzeichnet, dass** die mechanische Abtrennung mit einem Dekanter erfolgt.

**6.** Verfahren nach einem der vorangehenden Ansprüche, **dadurch gekennzeichnet, dass** die Trennschritte unter oxidationswidrigen Bedingungen durchgeführt werden, wie durch Zugabe eines Reduktionsmittels, wie Ascorbinsäure, Natriumbisulfit oder SO2; Arbeiten unter Schutz- gas und das Arbeiten in gasdichten Anlagen.

**Claims**

**1.** Process for the preparation of coagulated legume protein fractions selected from pea or field bean protein fractions of a molecular weight > 14, **characterized by**:

Providing fruit juice prepared by separating solids from comminuted fruits to release fruit juice from the cells, optionally with the addition of an aqueous solvent, and separating all or part of the fruit juice thus released containing all of the protein;

precipitating a high molecular weight legume fraction, the major amount of which has a molecular weight of > 116 kD < 600 kD according to the SDS-Page, by adjusting a pH in the range of the isoelectric point in the fruit juice of 5 - 7 while precipitating a fraction and mechanically separating the fraction thus precipitated; and completely or partially separating the fruit juice thus released, containing all the protein, from the solids; and precipitating a medium molecular weight coagulated legume protein fraction in the heat by treating the solution obtained from the separation of the high molecular weight legume protein fraction at pH 3 - 6 and between 60 - 90°C, preferably 65 - 80°C; and

mechanical separation of the medium molecular weight coagulated legume protein fraction with a molecular weight MG between 14 - 120 kD according to SDS-Page, whereas the center of gravity of the molecular weight distribution thereof is between 20 - 66 kD according to SDS-Page.

**2.** Process according to claim 1, **characterized by** washing the coagulated legume protein fraction thus obtained.

**3.** Process according to any one of the preceding claims, wherein the washing is carried out with water at a temperature from room temperature to 80°C, preferably at the temperature of precipitation.

**4.** Process according to claim 3, wherein the washing is carried out at a pH value in the range of the isoelectric point.

**5.** Process according to one of the preceding claims, **characterized in that** the mechanical separation is carried out with a decanter.

6. Process according to any one of the preceding claims, **characterized in that** the separation steps are carried out under conditions hindering oxidation, such as by adding a reducing agent, such as ascorbic acid, sodium bisuifite or SO2; working under protective gas and working in gas-tight systems.

**Revendications**

1. Procédé de production de fractions protéiques de légumineuses coagulées d'un poids moléculaire > 14, sélectionnées à partir de fractions protéiques de pois ou de féveroles, **caractérisé par** :

   une fourniture de jus de fruit produit par séparation de substances solides d'avec des fruits fragmentés en vue de libérer du jus de fruit à partir des cellules, avec adjonction de solvant aqueux le cas échéant, et séparation totale ou partielle du jus de fruit ainsi libéré, qui renferme l'intégralité de la protéine ;
   la précipitation d'une fraction de légumineuse à poids moléculaire élevé, dont la quantité majoritaire présente un poids moléculaire > 116 kD < 600 kD en conformité avec SDS-Page, par ajustement d'une valeur de pH de 5-7 dans la plage du point isoélectrique dans le jus de fruit, avec précipitation d'une fraction et séparation mécanique de la fraction ainsi précipitée ; et séparation totale ou partielle, d'avec les substances solides, du jus de fruit ainsi libéré qui renferme l'intégralité de la protéine ; et
   la précipitation, en milieu chaud, d'une fraction protéique de légumineuse coagulée de poids moléculaire moyen, par traitement de la solution obtenue à partir de la séparation de la fraction protéique de légumineuse à poids moléculaire élevé, en présence d'un pH 3-6 et entre 60-90 °C, préférentiellement 65 - 80 °C, et
   une séparation mécanique de ladite fraction protéique de légumineuse coagulée de poids moléculaire moyen présentant un poids moléculaire entre 14 - 120 kD en conformité avec SDS-Page, dont la dominante de la répartition du poids moléculaire se situe entre 20 - 66 kD en conformité avec SDS-Page.

2. Procédé selon la revendication 1, **caractérisé par** un lavage de la fraction protéique de légumineuse coagulée ainsi obtenue.

3. Procédé selon l'une des revendications précédentes, le lavage étant effectué à l'eau, à une température partant de la température ambiante et atteignant jusqu'à 80 °C, de préférence à la température de la précipitation.

4. Procédé selon la revendication 3, le lavage étant effectué à une valeur de pH située dans la plage du point isoélectrique.

5. Procédé selon l'une des revendications précédentes, **caractérisé par le fait que** la séparation mécanique est effectuée à l'aide d'un décanteur.

6. Procédé selon l'une des revendications précédentes, **caractérisé par le fait que** les étapes de séparation sont exécutées dans des conditions défavorables à l'oxydation, comme l'ajout d'un agent réducteur tel que l'acide ascorbique, le bisulfite de sodium ou le $SO_2$ ; travail sous gaz protecteur et travail dans des installations étanches aux gaz.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 2922247 A1

- DD 214527

### In der Beschreibung aufgeführte Nicht-Patentliteratur

- **O'KANE.** lehrt die wissenschaftliche Charakterisierung von Erbsenproteinen. *JOURNAL OF AGRICULTURAL AND FOOD CHEMISTRY,* 2004, vol. 52 (10), 3141-3148

- **CHAKRABORTY.** die Ultrazentrifugationstrennung von salzlöslichen Proteinen von 10 Leguminosen-früchten. *JOURNAL OF THE SCIENCE OF FOOD ANDAGRICULTURE,* 1979, vol. 30 (8), 766-711
- **SCHMANDKE.** *DIE NAHRUNG,* 1981, vol. 25 (4), 379-389